Europäisches Patentamt

**European Patent Office**

Office européen des brevets

(11) Veröffentlichungsnummer: **0 119 403**
**B1**

(12) **EUROPÄISCHE PATENTSCHRIFT**

(45) Veröffentlichungstag der Patentschrift:
07.06.89

(51) Int. Cl.⁴: **A 61 F 2/00**, A 61 L 27/00

(21) Anmeldenummer: **84100696.8**

(22) Anmeldetag: **24.01.84**

(54) **Bioaktives Implantatmaterial.**

(30) Priorität: **18.02.83 DE 3305572**

(43) Veröffentlichungstag der Anmeldung:
**26.09.84 Patentblatt 84/39**

(45) Bekanntmachung des Hinweises auf die Patenterteilung: **07.06.89 Patentblatt 89/23**

(84) Benannte Vertragsstaaten:
**AT BE CH DE FR GB IT LI**

(56) Entgegenhaltungen:
**FR-A- 2 230 598**
**FR-A- 2 301 488**
**GB-A- 2 080 281**
**CHEMICAL ABSTRACTS, Band 95, Nr. 22, November 1981, Seite 261, Nr. 191228z, Columbus, Ohio, US; T. KOKUBO et al.: "Preparation of fiber-oriented 3CaO.P2O5-CaO.SiO2-CaO.MgO.2SiO2 glass-ceramics"**
**CHEMICAL ABSTRACTS, Band 98, Nr. 16, 18. April 1983, Seite 368, Nr. 132266z, Columbus, Ohio, US; T. KOKUBO et al.: "Apatite- and wollastonite-containing glass-ceramics for prosthetic application"**

(73) Patentinhaber: **Wild Leitz GmbH**
**Ernst-Leitz-Strasse 30 Postfach 20 20**
**D-6330 Wetzlar 1 (DE)**

(72) Erfinder: **Deutscher, Klaus-Konrad, Dr.**
**Lerchenweg 20**
**D-6330 Wetzlar (DE)**
Erfinder: **Brömer, Heinz**
**Am Hundsrück 7**
**D-6330 Wetzlar (DE)**
Erfinder: **Franek, Henning, Dr.**
**Mühlenkopfstrasse 5**
**D-6333 Braunfels-Tiefenbach (DE)**

Jouve, 18, rue St-Denis, 75001 Paris, France

**Beschreibung**

Die Erfindung betrifft ein bioaktives Implantatmaterial. Alloplastische Implantate spielen im Vergleich zu homöologen oder heterologen Transplantaten eine immer größere Rolle. Das gilt besonders für den Bereich des Gelenkersatzes, wo artikulierende Flächen aus knöchernem Material mit entsprechenden Festigkeitsforderungen kaum zu realisieren sind. Die medizinische Forschung konzentriert sich auf die Entwicklung von Werkstoffen, die nach der Implantation komplikationslos einheilen. Wird andererseits das umgebende Wirtsgewebe durch das Implantatmaterial zu entzündlichen Reaktionen veranlaßt, dann führen solche Reaktionen letztendlich zur Abstoßung des Implantates. Ein solcher Werkstoff ist nicht biokompatibel und deshalb für Implantate ungeeignet.

Implantatmaterialien, die klinisch reaktionslos im Implantatbett verbleiben, werden als bioinerte Materialien bezeichnet. Neben einigen wenigen Legierungen ist besonders das Aluminiumoxid als bioinert zu bezeichnen. Die reaktionslos einheilenden bioinerten Implantatmaterialien sind bei mikroskopischer Beobachtung des grenzflächennahen Gewebes stets von einer dünnen, mehrlagigen, parallel zur Implantatoberfläche orientierten Zellmembran umschlossen. Die Dicke der Weichgewebskapsel kann als Maß für die Bioinertheit angesehen werden.

Für Implantationen in das knöcherne Skelettsystem kann die Tatsache der bindegewebigen Abkapselung des Implantates Schwierigkeiten für die Langzeitstabilität der Verankerung hervorrufen : Durch die Interposition der Weichgewebsschicht wird bei Belastung der Kraftfluß von einem Knochenteil über das Implantat zum lastaufnehmenden Knochen an der Grenzfläche : Implantat — Knochen gestört. Meist reagiert das knöcherne Gewebe auf eine unphysiologische Kraftflußeinleitung mit Umbauvorgängen, die zu einer Verbreiterung der Bindegewebskapsel bis hin zur klinischen Lockerung des Implantates führt.

Den Nachteil der bindegewebigen Abkapselung von bioinerten Implantatmaterialien weisen die bioaktiven Implantatmaterialien nicht auf. Ihnen gegenüber reagiert das knöcherne Implantatmaterial nicht mit der Bildung einer Bindegewebsmembran. Es findet vielmehr eine bindegewebsfreie, mechanisch belastbare Verbindung zwischen Knochen und bioaktivem Material statt.

Bioaktive Implantatmaterialien sind bekannt. So beschreibt Hench 1971 erstmals den bindegewebsfreien Verbund des knöchernen Gewebes mit einem « Bioglas » genannten Material auf Silikophosphatbasis mit 25 %-igem $Na_2O$-Gehalt. In der DE-PS 2 326 100 wird ferner eine Glaskeramik beschrieben, die aus einem Grundglas mit einem reduzierten $Na_2O$-Gehalt von 2,7-20,0 Gew.-% keramisiert wird. Diese Zusammensetzung zeichnet sich dadurch aus, daß infolge der Oberflächenlöslichkeit nicht nur $Na^+$- und $Ca^{+2+}$-Ionen in das umgebende Wirtsgewebe eingegeben werden, sondern durch Zusatz von MgO und $K_2O$ in das Grundglas auch $K^+$- und $Mg^{+2+}$-Ionen als Antagonisten abgegeben werden, wodurch das physiologische Ionengleichgewicht in der Implantatumgebung nicht nachhaltig gestört wird.

Aus Chemical Abstracts, Band 95, Nr. 22, November 1981, Seite 261, Nr. 191228z, Columbus, Ohio, Us ; T. Kokubo et al : « Preparation of fiber-oriented $3CaO \cdot P_2O_5$-$CaO \cdot SiO_2$-$CaO \cdot MgO \cdot 2SiO_2$ glass-ceramics », ist eine Grundglas-Zusammensetzung bekannt, die neben den Komponenten $SiO_2$, $P_2O_5$ und CaO als weitere essentielle Komponente MgO enthält. Der dort beschriebene Temperaturgradientenprozeß zur Durchführung der Keramisierungsbehandlung führt zu einer ß-Wollastonit und Apatit enthaltenen Glaskeramik.

Neben den bioaktiven Gläsern und bioaktiven Glaskeramiken sind auch Calciumphosphat-Keramiken und Apatit als bioaktive Implantatwerkstoffe beschrieben worden. Aufgrund der hohen Löslichkeit der Calciumphosphat-Keramiken werden diese meist noch als poröse Implantate als temporäre Platzhalter für die Auffüllung von Zysten oder im Falle des Apatits zur Augmentation des Unterkiefers durch Füllung der Alveolen mit entsprechenden zylindrischen oder konischen Implantatkörpern vorgeschlagen. In vielen Fällen wird den in Lösung gehenden Ionen eine knochenwachstumsinduzierende Wirkung nachgesagt.

Prothesen aus bioaktivem glaskeramischen Material sind als Vollmaterial bei lastfreier Applikation weit verbreitet. Für hochbelastete, orthopädische Prothesen reicht die Biegebruchfestigkeit von 15 kp/mm² nicht aus.

Es wurde daher bereits in der DE-PS 2 546 824 eine Beschichtung von hochbelastbarem metallischen Prothesenmaterial im Schaftbereich bzw. im Kontaktbereich zu Knochen mit bioaktivem Material vorgeschlagen, wobei das bioaktive Material nicht als geschlossene Schicht, sondern als in eine biokompatible Emailschicht eingebettetes Granulat durch diese haftvermittelnde Emailschicht fest mit der Metalloberfläche verbunden ist. Tierexperimente haben gezeigt, daß mit diesem Prinzip eine Bioaktivierung von metallischem Implantatmaterial möglich ist.

Die bioaktive Glaskeramik wird — wie tierexperimentelle Langzeituntersuchungen zeigen — in das physiologische Bone-remodeling einbezogen. Bei diesem permanenten Umbauvorgang werden knöcherne Areale zu Weichgewebe (Havers'sche Bereiche), das der Versorgung des wachsenden Knochens dient, umgebaut und umgekehrt Weichgewebe mineralisiert. Dieser Prozess läuft auch an der Grenzfläche des Knochens zum Implantat. Dabei wird ein massives bioaktives Implantat aus Glaskeramik nur unwesentlich durch die gegenüber dem Weichgewebe höhere Oberflächenlöslichkeit beeinflußt. Für die bis zu 50 μm kleinen bioaktiven Partikel kann die höhere Oberflächenlöslichkeit gegenüber der sich während des Bone-remodeling bildenden Weichgewebes ein die Langzeitstabilität begrenzender Faktor darstellen.

Es ist daher die Aufgabe der vorliegenden Erfindung, ein bioaktives Implantatmaterial mit reduzierter Oberflächenlöslichkeit unter Beibehaltung der essentiellen Eigenschaft der Bioaktivität zu schaffen.

Dies Aufgabe wird erfindungsgemäß durch ein bioaktives Implantatmaterial gelöst, welches aus 8 bis 60 Gew.-% $SiO_2$, 3 bis 40 Gew.-% $P_2O_5$ und aus 25 bis 70 Gew.-% CaO besteht. Bevorzugte Zusammensetzungen bestehen aus 46,0 bzw. 22,6 Gew.-% $SiO_2$, 11,5 bzw. 24,4 $P_2O_5$ und 42,5 bzw. 53,0 Gew.-% CaO. Es ist besonders vorteilhaft, das Implantatmaterial als bioaktive Glaskeramik auszubilden, deren Kristallin-Anteil aus zwei diskreten Phasen besteht. Nach einer besonderen Ausführungsform der vorliegenden Erfindung bestehen diese Phasen aus Kristalliten von Apatit und Wollastonit. Auch ist es insbesondere möglich, daß es sich bei den Kristalliten um die jeweiligen Hochtemperatur-Modifikationen von Apatit und Wollastonit handelt. Die Aufgabe wird weiterhin durch ein Verfahren zur Herstellung eines bioaktiven Implantatmaterials derart gelöst, daß ein Gemenge aus $SiO_2$, $Ca(PO_3)_2$ und CaO mit dem im Hauptanspruch angegebenen oxidischen Zusammensetzungsbereichen zunächst zu einem Grundglas erschmolzen und danach einem Temperatur-Zeit-Keramisierungsprozeß unterworfen wird, der aus den folgenden Verfahrensstufen besteht: Keimbildungs- und nachfolgende Kristallisationsphase für Apatit, Keimbildungs- und nachfolgende Kristallisationsphase für Wollastonit, Überführen der kristallinen Phasen in ihre jeweiligen Hochtemperatur-Modifikationen, Abkühlphase. Besonders vorteilhafte Gemengezusammensetzungen bestehen aus 46,0 bzw. 22,6 Gew.-% $SiO_2$, 16,0 bzw. 34,0 Gew.-% $Ca(PO_3)_2$ und 38,0 bzw. 43,4 Gew.-% CaO.

In den nachfolgenden Tabellen sind Einzelbeispiele angegeben, wobei Tabelle I erfindungsgemäße Implantatmaterial-Zusammensetzungen in oxidischer Form enthält, während die Tabelle II die entsprechenden Verbindungen angibt, die als zusammengemischte chemische Komponenten das Gemenge zur Erzeugung eines Grundglases bilden.

Tabelle I (Gew.-%)

| Nr. | $SiO_2$ | $P_2O_5$ | CaO |
|---|---|---|---|
| 1 | 60,0 | 3,6 | 36,4 |
| 2 | 35,0 | 3,6 | 61,4 |
| 3 | 46,0 | 11,5 | 42,5 |
| 4 | 38,9 | 11,0 | 50,1 |
| 5 | 22,6 | 24,4 | 53,0 |
| 6 | 8,0 | 37,3 | 54,7 |

Tabelle II (Gew.-%)

| Nr. | $SiO_2$ | $Ca(PO_3)_2$ | CaO |
|---|---|---|---|
| 1 | 60,0 | 5,0 | 35,0 |
| 2 | 35,0 | 5,0 | 60,0 |
| 3 | 46,0 | 16,0 | 38,0 |
| 4 | 38,9 | 15,3 | 45,8 |
| 5 | 22,6 | 34,0 | 43,4 |
| 6 | 8,0 | 52,0 | 40,0 |

Der Vorteil des erfindungsgemäßen Materials besteht insbesondere darin, daß die Langzeitstabilität eines derartigen Implantates in vivo erheblich verbessert werden konnte.

Die an Bedeutung immer mehr zunehmende Forderung des Osteochirurgen nach einem bioaktiven Implantatwerkstoff, aus dem ohne Einbuße der unverzichtbaren Eigenschaft der Bioaktivität ein permanent in situ verbleibendes — also dauerhaft formschlüssig positionierbares — Implantat herstellbar ist, konnte mit dem erfindungsgemäßen glaskeramischen « Verbundwerkstoff » in bislang nicht gekanntem Maße realisiert werden.

Überraschenderweise hat sich gezeigt, daß die Eigenschaft die Bioaktivität — nämlich Ausbildung einer belastungsfähigen bindegewebsfreien Verbindung zwischen Knochen und alloplastischem Material

3

— nicht an die Ionenabgabe des Implantatmaterials infolge Oberflächenlöslichkeit gebunden ist. Da eine Belastung des Organismus mit einer Ionenart ausgeschlossen werden kann, ist es auch nicht mehr notwendig, die entsprechenden Antagonisten anzubieten.

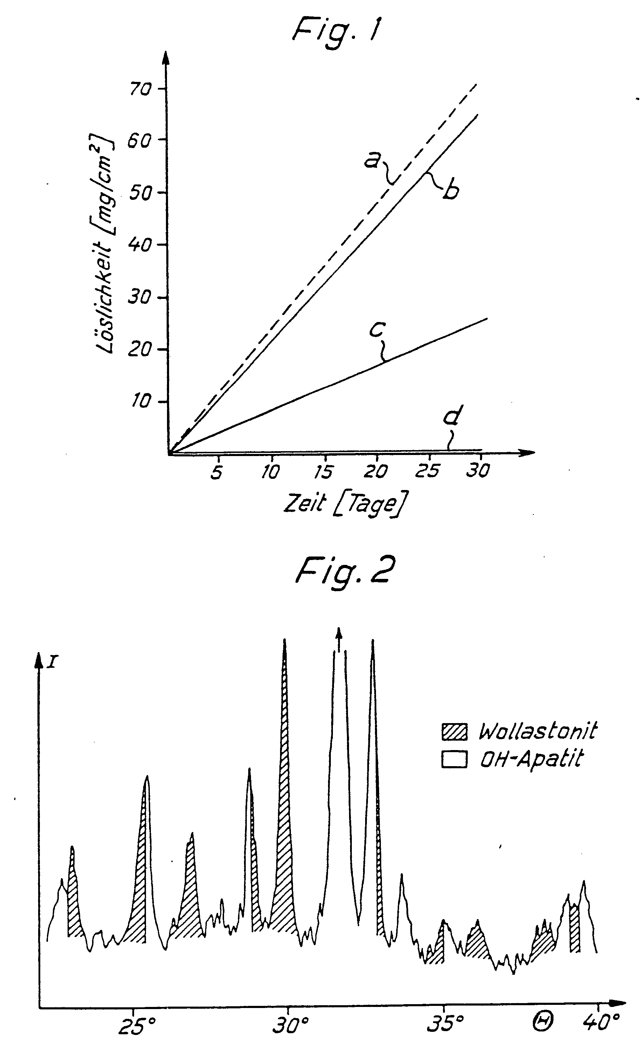

Die Keramisierung der Grundgläser wird in der Weise durchgeführt, daß zunächst die Keimbildung für die Apatitphase induziert wird. Daran schließt sich bei höherer Temperatur die Bildung der Wollastonitphase an. Die durch die Bildung der 2. Kristallphase in dem ternären System verursachte Reduzierung der Löslichkeit ist signifikant. Dies wird in Fig. 1 veranschaulicht. In dieser graphischen Darstellung wird die in vitro-Löslichkeit bioaktiver Glaskeramiken in einem wässrigen Medium ($p_H = 7$) bei einer Temperatur von 90 °C angegeben, wobei die Kurve a einer bekannten bioaktiven Glaskeramik zuzuordnen ist, während die Kurven b, c und d die Löslichkeitsverhältnisse bei einem erfindungsgemäßen Material bzw. dessen Vorstufen wie folgt kennzeichnen :

Kurve b : (glasiges) Grundglas
Kurve c : partiell keramisierte Glaskeramik (1 kristalline Phase in glasiger Matrix)
Kurve d : total keramisierte Glaskeramik (2 kristalline Phasen in glasiger Matrix)

Auf der Abszisse ist die Einwirkungszeit in Tagen und auf der Ordinate die Löslichkeit des Materials in Milligramm pro Oberflächeneinheit [$cm^2$] aufgetragen.

In Fig. 2 wird ein Teilbereich (Glanzwinkel $\Theta$ : zwischen 21° und 40°) eines Röntgendiffraktometer-Diagramms des Beispiels 3 von Tabelle I gezeigt. Auf der Ordinate ist die Reflexintensität in willkürlichen Einheiten aufgetragen. Diese Zählrohrgoniometer-Aufnahme wurde bei folgenden Bedingungen durchgeführt :

| | |
|---|---|
| Röntgenstrahlung : | $CuK_\alpha$ |
| Wellenlänge : | 1.5418 Å |
| Filter : | Ni |
| Spannung : | 36 kV. |

Sie zeigt die für eine Identifizierung der Kristallin-Anteile spezifischen Intensitäts-Peaks. Die für Wollastonit ($CaSiO_3$) charakteristischen Intensitätsverteilungen gemäß ASTM-Karteikarte Nr. 29-372 wurden schraffiert dargestellt, während die für Hydroxylapatit ($Ca_5(PO_4)_3OH$) typischen Intensitätsmaxima sich ohne besondere Hervorhebung aus dem Kurvenverlauf ergeben, wobei der Haupt-Peak bei $\Theta = 31,6°$ noch um ca. 1/3 höher ist, als in Fig. 2 dargestellt. Die Identifizierung des Apatits gelang mit Hilfe der ASTM-Karteikarte Nr. 9-432.

Die vorliegende röntgenographische Phasenanalyse zeigt, daß das erfindungsgemäße, voll auskeramisierte Material (Beispiel 3) neben einem röntgenamorphen (glasigen) Matrix-Anteil zwei diskreten kristalline Phasen, nämlich Apatit und Wollastonit, aufweist. Dieses End-Produkt kann mithin als bioaktives glaskeramisches « Verbundmaterial » bezeichnet werden.

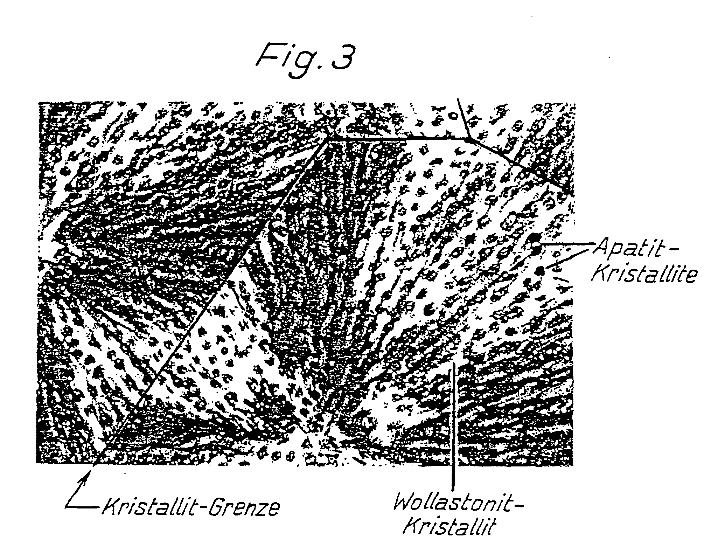

Ein mikroskopisches Dünnschliffbild (mit gekreuzten Nicols) eines derartigen Werkstoffes wird in Fig. 3 gezeigt. Man erkennt einzelne großflächige, sphärolithische Wollastonit-Kristallite und ihre linear verlaufenden Kristallit-Korngrenzen (die Korngrenzen wurden in der Abbildung verstärkt dargestellt), sowie eine Vielzahl kleinerer, isometrischer Apatit-Kristalle.

**Patentansprüche** (für die Vertragsstaaten : BE, CH, DE, FR, GB, IT, LI)

1. Bioaktives, glaskeramisches Implantatmaterial, dadurch gekennzeichnet, daß es aus
8-60 Gew.-% $SiO_2$
3-40 Gew.-% $P_2O_5$ und
25-70 Gew.-% CaO
besteht.

2. Implantatmaterial nach Anspruch 1, dadurch gekennzeichnet, daß es aus
46,0 Gew.-% $SiO_2$
11,5 Gew.-% $P_2O_5$ und
42,5 Gew.-% CaO
besteht.

3. Implantatmaterial nach Anspruch 1, dadurch gekennzeichnet, daß es aus
22,6 Gew.-% $SiO_2$
24,4 Gew.-% $P_2O_5$
53,0 Gew.-% CaO
besteht.

4. Implantatmaterial nach einem der Ansprüche 1-3, dadurch gekennzeichnet, daß es eine Glaskeramik ist, deren kristalliner Anteil aus zwei diskreten Phasen besteht.

5. Implantatmaterial nach Anspruch 4, dadurch gekennzeichnet, daß die Phasen aus Kristalliten von Apatit und Wollastonit bestehen.

6. Implantatmaterial nach einem der Ansprüche 4 und 5, dadurch gekennzeichnet, daß die diskreten Phasen aus ihren jeweiligen Hochtemperatur-Modifikationen bestehen.

7. Verfahren zur Herstellung eines Materials nach Anspruch 1, dadurch gekennzeichnet, daß ein Gemenge aus $SiO_2$, $Ca(PO_3)_2$ und CaO mit dem im Anspruch 1 angegebenen oxidischen Zusammensetzungsbereich zu einem Grundglas erschmolzen und danach einem Keramisierungsprozeß mit den Verfahrensstufen

(a) Keimbildungsphase für Apatit
(b) Kristallisationsphase für Apatit
(c) Keimbildungsphase für Wollastonit
(d) Kristallisationsphase für Wollastonit
(e) Erzeugung von Hochtemperatur-Modifikationen beider kristalliner Phasen
(f) Abkühlphase

unterworfen wird.

8. Verfahren nach Anspruch 8, dadurch gekennzeichnet, daß das Gemenge aus

46,0 Gew.-% $SiO_2$
16,0 Gew.-% $Ca(PO_3)_2$ und
38,0 Gew.-% CaO

besteht.

9. Verfahren nach Anspruch 8, dadurch gekennzeichnet, daß das Gemenge aus

22,6 Gew.-% $SiO_2$
34,0 Gew.-% $Ca(PO_3)_2$ und
43,4 Gew.-% CaO

besteht.


**Patentansprüche** (für den Vertragsstaat AT)

1. Verfahren zur Herstellung eines bioaktiven, glaskeramischen Implantatmaterials, dadurch gekennzeichnet, daß ein Gemenge aus $SiO_2$, $Ca(PO_3)_2$ und CaO in einem gegenseitigen Verhältnis der oxidischen Zusammensetzung von

8-60 Gew.-% $SiO_2$
3-40 Gew.-% $P_2O_5$ und
25-70 Gew.-% CaO

zu einem Grundglas erschmolzen und danach einem Keramisierungsprozeß unterworfen wird.

2. Verfahren nach Anspruch 1, dadurch gekennzeichnet, daß im Keramisierungsprozeß die Verfahrensstufen

a) Keimbildungsphase für Apatit
b) Kristallisationsphase für Apatit
c) Keimbildungsphase für Wollastonit
d) Kristallisationsphase für Wollastonit
e) Erzeugung von Hochtemperatur-Modifikationen beider kristalliner Phasen
f) Abkühlphase

durchlaufen werden.

3. Verfahren nach Anspruch 1 oder 2, dadurch gekennzeichnet, daß das Gemenge aus

46,0 Gew.-% $SiO_2$
16,0 Gew.-% $Ca(PO_3)_2$ und
38,0 Gew.-% CaO

hergestellt wird.

4. Verfahren nach Anspruch 1 oder 2, dadurch gekennzeichnet, daß das Gemenge aus

22,6 Gew.-% $SiO_2$
34,0 Gew.-% $Ca(PO_3)_2$ und
43,4 Gew.-% CaO

hergestellt wird.

5. Verfahren nach einem der Ansprüche 1 bis 4, dadurch gekennzeichnet, daß eine Glaskeramik hergestellt wird, deren kristalliner Anteil aus zwei diskreten Phasen besteht.

6. Verfahren nach Anspruch 5, dadurch gekennzeichnet, daß die Phasen durch Kristallite von Apatit und Wollastonit gebildet werden.

7. Verfahren nach Anspruch 5 oder 6, dadurch gekennzeichnet, daß die diskreten Phasen durch ihre jeweiligen Hochtemperatur-Modifikationen gebildet werden.


**Claims**

1. Bio-active glass-ceramic implant material, characterised thereby, that it consists of

8 to 60 % by weight of $SiO_2$

3 to 40 % by weight of $P_2O_5$ and
25 to 70 % by weight of CaO.

2. Implant material according to claim 1, characterised thereby, that it consists of
46.0 % by weight of $SiO_2$
11.5 % by weight of $P_2O_5$ and
42.5 % by weight of CaO.

3. Implant material according to claim 1, characterised thereby, that it consists of
22.6 % by weight of $SiO_2$
24.4 % by weight of $P_2O_5$ and
53.0 % by weight of CaO.

4. Implant material according to one of the claims 1 to 3, characterised thereby, that it is a glass-ceramic, the crystalline component of which consists of two discrete phases.

5. Implant material according to claim 4, characterised thereby, that the phases consist of crystallites of apatite and wollastonite.

6. Implant material according to one of the claims 4 and 5, characterised thereby, that the discrete phases consist of their respective high temperature modifications.

7. Method for the manufacture of a material according to claim 1, characterised thereby, that a batch of $SiO_2$, $Ca(PO_3)_2$ and CaO with the oxidic composition range stated in claim 1 is molten to a basic glass and thereafter subjected to a ceramicising process with the method stages

a) seed formation phase for apatite,
b) crystallisation phase for apatite,
c) seed formation phase for wollastonite,
d) crystallisation phase for wollastonite,
e) production of high temperature modifications of both crystalline phases and
f) cooling phase.

8. Method according to claim 8, characterised thereby, that the batch consists of
46.0 % by weight of $SiO_2$
16.0 % by weight of $Ca(PO_3)_2$ and
38.0 % by weight of CaO.

9. Method according to claim 8, characterised thereby, that the batch consists of
22.6 % by weight of $SiO_2$
34.0 % by weight of $Ca(PO_3)_2$ and
43.4 % by weight of CaO.

**Revendications**

1. Matériau implantable vitrocéramique bioactif, caractérisé en ce qu'il consiste en
8-60 % en poids de $SiO_2$
3-40 % en poids de $P_2O_5$ et
25-70 % en poids de CaO

2. Matériau implantable selon la revendication 1, caractérisé en ce qu'il consiste en
46,0 % en poids de $SiO_2$
11,5 % en poids de $P_2O_5$ et
42,5 % en poids de CaO

3. Matériau implantable selon la revendication 1, caractérisé en ce qu'il consiste en
22,6 % en poids de $SiO_2$
24,4 % en poids de $P_2O_5$
53,0 % en poids de CaO

4. Matériau implantable selon l'une des revendications 1-3, caractérisé en ce qu'il s'agit d'une vitrocéramique dont la fraction cristalline consiste en deux phases distinctes.

5. Matériau implantable selon la revendication 4, caractérisé en ce que les phases consistent en des cristallites d'apatite et de wollastonite.

6. Matériau implantable selon l'une des revendications 4 et 5, caractérisé en ce que les phases discrètes consistent en leurs modifications à haute température respectives.

7. Procédé pour la fabrication d'un matériau selon la revendication 1, caractérisé en ce qu'un mélange de $SiO_2$, $Ca(PO_3)_2$ et CaO ayant la gamme de composition d'oxydes indiquée à la revendication 1 est transformé par fusion en un verre de base puis est soumis à un processus de céramisation comprenant les étapes suivantes

(a) Phase de formation de germes pour l'apatite
(b) Phase de cristallisation pour l'apatite
(c) Phase de formation de germes pour la wollastonite
(d) Phase de cristallisation pour la wollastonite
(e) Production des modifications à haute température des deux phases cristallines
(f) Phase de refroidissement

8. Procédé selon la revendication 7, caractérisé en ce que le mélange consiste en
46,0 % en poids de $SiO_2$
16,0 % en poids de $Ca(PO_3)_2$ et
38,0 % en poids de CaO

9. Procédé selon la revendication 8, caractérisé en ce que le mélange consiste en
22,6 % en poids de $SiO_2$
34,0 % en poids de $Ca(PO_3)_2$ et
43,4 % en poids de CaO.

## Fig. 1

## Fig. 2

## Fig. 3

Apatit-Kristallite

Kristallit-Grenze

Wollastonit-Kristallit